# EUROPÄISCHE PATENTSCHRIFT

(11) **EP 2 421 614 B1**
(45) Veröffentlichungstag und Bekanntmachung des Hinweises auf die Patenterteilung: **03.04.2013**
(21) Anmeldenummer: 10713858.8
(22) Anmeldetag: 16.04.2010
(51) Int. Cl.: A61Q 17/04, A61K 8/34, A61K 8/27, A61K 8/29, A61K 8/35, A61K 8/44, A61K 8/49

(54) **Kosmetische oder dermatologische Zubereitung**
Cosmetic or dermatological preparation
Préparation cosmétique ou dermatologique

(30) Priorität: 20.04.2009 EP 09005531
(43) Veröffentlichungstag der Anmeldung: 29.02.2012
(73) Patentinhaber: Dr. Straetmans GmbH, 22143 Hamburg (DE)
(72) Erfinder: NAZAROVA, Daria, 22159 Hamburg (DE); SCHOLZE, Jessica, 21435 Stelle (DE); JÄNICHEN, Jan, 22149 Hamburg (DE); PETERSEN, Wilfried, 22143 Hamburg (DE); SALMINA-PETERSEN, Manuela, 22143 Hamburg (DE)
(74) Vertreter: Glawe, Delfs, Moll
(86) Internationale Anmeldenummer: PCT/EP2010/002356
(87) Internationale Veröffentlichungsnummer: WO 2010/121759

(56) Entgegenhaltungen:
- EP-A2- 1 859 779
- DE-A1- 19 703 471
- DE-A1-102007 017 440
- "Emollients in Personal Care for Solubilizing Organic UV Absorbers" IP.COM JOURNAL, IP.COM INC., WEST HENRIETTA, NY, US, 22. Februar 2006 (2006-02-22), XP013112931 ISSN: 1533-0001

## Beschreibung

Die Erfindung betrifft kosmetische oder dermatologische Zubereitungen wie bspw. Sonnenschutzzubereitungen.

Dem Schutz der menschlichen Haut vor schädigenden UV-Strahlen ist nicht nur durch die zunehmende Ausdünnung der Ozonschicht in einigen Teilen der Erde sondern auch durch das Freizeitverhalten des Menschen in den vergangenen Jahren eine zunehmende Bedeutung zugewachsen.

Während der sehr kurzwellige UV-Bereich von < 290 nm normalerweise von der atmosphärischen Ozonschicht absorbiert wird, gelangen die längerwelligen UV-B Strahlen mit einer Wellenlänge von 290 - 320 nm sowie die UV-A Strahlen mit einer Wellenlänge von 320 - 400 nm relativ ungehindert an die Erdoberfläche und können je nach Einwirkungsdauer und -frequenz auf der Haut temporäre aber auch bleibende Schädigungen hervorrufen. So führt bereits eine kurzzeitige Einwirkung von UV-Strahlung von ca. 310 nm rasch zu einem schmerzhaften Sonnenbrand während eine längere Einwirkung langwelliger UV-A-Strahlung in den tieferen Hautschichten phototoxische Reaktionen hervorrufen kann und über eine Schädigung des Bindegewebes die Haut schneller altern lässt. Jedoch kann nicht nur das Erscheinungsbild der Haut unter extensiver UV-Einwirkung in Mitleidenschaft gezogen werden, auch maligne Zellveränderungen, verbunden mit der Ausbildung von Melanomen werden UV-induzierten Schädigungen des genetischen Materials der Hautzellen zugeschrieben. Lichtschutzprodukten wächst somit eine große Bedeutung bei der Gesunderhaltung der Haut zu.

Moderne Lichtschutzprodukte bedienen sich Filtersystemen, welche in der Lage sind, UV-Strahlen auf der Hautoberfläche zu absorbieren oder zu reflektieren. Dem Fachmann ist bekannt, dass man zwischen chemisch und physikalisch wirkenden UV-Filtern unterscheiden kann. Die Funktionsweise chemischer UV-Filter basiert darauf, dass diese in der Lage sind, energiereiche UV-Strahlung zu absorbieren und dabei in einen angeregten Zustand übergehen. Aus diesem relaxieren die angeregten Spezies anschließend unter inkrementärer Abgabe der aufgenommenen Energie wieder in ihren Grundzustand.

Physikalisch wirkende UV-Filter, basierend auf anorganischen Pigmenten wie z.B. TiO₂ oder ZnO hingegen reflektieren die UV-Strahlung und verhindern auf diese Weise deren Eindringen in die Haut. Da sich chemische und physikalische UV-Filter in vielen Fällen ergänzen, wird in modernen Sonnenschutzprodukten vielfach auf Kombinationen aus beiden Systemen zurückgegriffen. Kosmetische Lichtschutzprodukte, welche den Anforderungen von Naturkosmetik entsprechen sollen, dürfen gemäß den formulierten Standards für derartige Produkte keine chemischen UV-Filter enthalten. Ihr UV-Schutz basiert ausschließlich auf der Verwendung physikalischer UV-Filter.

Die Fähigkeit von Lichtschutzprodukten, UV-Strahlen am Eindringen in die Haut zu hindern wird durch deren Sonnenschutzfaktor (SPF) ausgedrückt. In früheren Jahren wurde dieser Wert in in-vivo Versuchen ermittelt, indem das Auftreten von Hautrötungen bei einer definierten Bestrahlungsmenge und -dauer bestimmt wurde. Da die mit Hautrötungen verbundenen entzündlichen Prozesse in der Haut jedoch eher durch die Einwirkung kurzwelliger UV-B-Strahlen ausgelöst werden, kann durch diese Methode nicht das Maß für den Schutz gegen UV-A Strahlungen ermittelt werden. Da die Wirkung von UV-A Strahlen in der Haut wie oben beschrieben, jedoch nicht minder gefährlich ist, wurden in der jüngeren Vergangenheit ergänzende in-vitro Methoden zur Bestimmung der UVA-Absorption entwickelt, die diesem Mangel am Stand der Technik Abhilfe geschaffen hat. Sie sollen sicher stellen, dass Lichtschutzprodukte über den gesamten UV-Bereich eine schützende Wirkung besitzen.

Zur Entfaltung einer optimalen Wirkung beider, der chemischen UV-Filter und der anorganischen Pigmente, ist deren homogene Verteilung auf der Hautoberfläche eine wichtige Vorraussetzung. Darüber hinaus ist eine geringe Penetrationsneigung der chemischen UV-Filter in die Haut von großer Bedeutung, um Reaktionen der angeregten, häufig radikalischen Spezies in tieferen Hautschichten zu verhindern. Diese Parameter werden vor allen von den in der Formulierung verwendeten Ölkomponenten gesteuert.

Der Fachmann verwendet daher zur Herstellung von Sonnenschutzprodukten solche Ölkomponenten, die mehrere Eigenschaften in sich vereinen. So müssen sie über einen breiten Temperaturbereich Lichtschutzfilter für verschiedene UV-Bereiche gut lösen, anorganische Pigmente stabil dispergieren und zum Zweck einer homogenen Verteilung der Lichtschutzfaktoren auf der Haut über ein gutes Spreitvermögen verfügen.

Darüber hinaus werden noch weitere Anforderungen an Ölkomponenten für moderne Lichtschutzprodukte formuliert. So spielen die kosmetischen Eigenschaften der Formulierung eine wichtige Rolle bei der Kontinuität der Anwendung des Produktes durch den Verbraucher. Damit richtet sich die Qualität des Schutzes gegen schädliche UV-Strahlung auch direkt nach deren Anwenderfreundlichkeit. Schließlich ist die Gewinnung von Rohstoffen für dermatologische und kosmetische Produkte in den letzten Jahren in den Fokus der Aufmerksamkeit gerückt. Hierbei ist ein starker Trend hin zu Rohstoffen aus nachwachsenden Rohstoffen zu beobachten gewesen.

Der Erfindung liegt die Aufgabe zu Grunde, eine Zubereitung der eingangs genannten Art zur Verfügung zu stellen, die eine wirkungsvolle und anwendungsfreundliche Formulierung chemischer und/oder physikalischer UV-Filter in diesen Zubereitungen ermöglicht.

Überraschenderweise wurde nun gefunden, dass Ester einer C8-C16-Fettsäure und Isoamylalkohol wie bspw. Isoamyl Laurate für eine Reihe von gebräuchlichen chemischen und physikalischen UV-Filtern diese Anforderungen in hervorragender Weise erfüllen und somit eine gute Lösung für die Herausforderungen an die Qualität von Ölkomponenten für Lichtschutzformulierungen bieten.

Gegenstand der Erfindung ist eine kosmetische oder dermatologische Zubereitung, die enthält:
a) einen oder mehrere chemische Lichtschutzfilter ausgewählt aus der Gruppe bestehend aus 2,4,6-Trianilino-p-(carbo-2'-ethylhexyl-1'oxy)-1,3,5-triazin (Ethylhexyl Triazon), 1-(4-tert-butylphenyl)-3-(4-methoxyphenyl)-propan-1,3-dion (Butyl Methoxydibenzoyl-methan), Bis-octyloxyphenol-methoxyphenyl-triazin (Bis-Ethylhexyloxyphenol Methoxyphenyl Triazine) und 2-[-4-(Diethylamino)-2-hydroxybenzoyl] benzoesäurehexylester (Diethylamino Hydroxybenzoyl Hexyl Benzoate); und/oder einen oder mehrere Lichtschutzfilter ausgewählt aus der Gruppe bestehend aus oberflächlich behandelten oder unbehandelten Pigmenten des Titandioxid oder Zinkoxid;
b) wenigstens einen Ester einer C8-C16-Fettsäure und Isoamylalkohol.

Die vorliegende Erfindung betrifft somit kosmetische und dermatologische Zubereitungen, insbesondere Sonnenschutzprodukte, mit einem Gehalt eines Esters einer C8-C16-Fettsäure und Isoamylalkohol (bevorzugt Isoamyl Laurate) und einem oder mehreren chemischen UV-Filtern aus der Gruppe von Ethylhexyl Triazon, Butyl Methoxydibenzoylmethan, Diethylamino Hydroxybenzoyl Hexyl Benzoate und Bis-Ethylhexyloxyphenol Methoxyphenyl Triazine und/oder einem oder mehreren physikalischen UV-Filtern aus der Gruppe unmodifizierter oder oberflächlich modifizierte Zinkoxid- und/oder Titandioxid Pigmente.

Chemische UV-Filter gehören strukturell gesehen zur Verbindungsklasse der Aromaten, deren Absorptionsspektrum durch Substituenten und/oder Heteroatome in den Wellenlängenbereich des UV-Lichtes verschoben wurde. Eine Herausforderung bei der Formulierung der chemischen UV-Filter ist deren bis auf wenige Ausnahmen nur geringe Wasserlöslichkeit und ausgeprägte Kristallisationsneigung. Letztere Eigenschaft hat häufig auch eine begrenzte Öllöslichkeit zur Folge, was die Einsatzkonzentration chemischer UV-Filter und damit den Lichtschutzfaktor der betreffenden Formulierung limitiert.

Erschwert wird das Problem dadurch, dass die Absorptionsmaxima der chemischen UV-Filter nicht den gesamten Bereich schädlicher UV-A- und UV-B-Strahlung abdecken. Diese Tatsache erfordert vielfach den Einsatz von Gemischen von UV-Filtern mit unterschiedlichen Absorptionsmaxima, um einen möglichst effektiven Breitbandschutz der Haut vor den schädlichen Effekten der UV-Strahlung durch ein Sonnenschutzprodukt zu gewährleisten. Besonders vorteilhafte UV-Filter, die sich durch gute Lichtschutzeigenschaften auszeichnen und die in der jüngsten Vergangenheit breiten Einsatz gefunden haben, sind z.B. der UV-B-Filter 2,4,6-Trianilino-p-(carbo-2'-ethylhexyl-1'oxy)-1,3,5-triazin (INCI: Ethylhexyl Triazon), welcher von der BASF unter dem Handelsnamen Uvinul T 150 vertrieben wird, der UV-A-Filter 1-(4-tert-butylphenyl)-3-(4-methoxyphenyl)-propan-1,3-dion (INCI: Butyl Methoxydibenzoylmethan), welcher z.B. unter dem Handelsnamen Parsol 1789 von der Fa. DSM angeboten wird oder der 2-[-4-(Diethylamino)-2-hydroxybenzoyl] benzoesäurehexylester (INCI: Diethylamino Hydroxybenzoyl Hexyl Benzoate) welcher unter dem Handelsnamen Uvinul A Plus von der BASF vermarktet wird sowie das Bis-Octyloxy-phenolmethoxyphenyltriazin (INCI: Bis-Ethylhexyloxyphenol Methoxyphenyl Triazine), ein Breitbandfilter, der z.B. unter dem Handelsnamen Tinosorb S von der Fa. Ciba Geigy (EP0775698 B1) angeboten wird. Dem Fachmann ist bekannt, dass diese UV-Filter besonders hohe Anforderungen an das Lösungsvermögen der verwendeten Ölkomponenten stellen.

Komplementiert wird die Lichtschutzwirkung der chemischen UV-Filter durch anorganische Pigmente wie z.B. Titandioxid oder Zinkoxid, welche in feinst vermahlener Form die UV-Strahlung reflektieren. Um deren Kompatibilität mit den anderen Bestandteilen von Lichtschutzformulierungen zu erhöhen, werden diese Pigmente vielfach oberflächlich hydrophobiert. Ölkomponenten, welche die Einarbeitung derartiger Pigmente fördern, müssen vorteilhafte chemisch-physikalische Eigenschaften besitzen. So sollten sie z.B. eine niedrige Oberflächenspannung bei gleichzeitig hoher Polarität besitzen und dadurch über eine gute Benetzfähigkeit verfügen.

Dem Stand der Technik entsprechend werden diese Anforderungen derzeit von Ölen verschiedener Strukturen gelöst. Beispielhaft seien hier die Gruppe der linearen Alkylbenzoate, der Ester von 1,3-Butylenglycol oder 1,3-Propylenglycol sowie der Ester von Dicarbonsäuren genannt.

Aus der Gruppe der linearen Alkylbenzoate findet das C12-15 Alkylbenzoat, welches unter den Markennamen Finsolv TN oder Cetiol AB angeboten wird, breite Verwendung. C12-15 Alkylbenzoat stellt zwar eine gute Möglichkeit zur Lösung der UV-Filter und dem Dispergieren von Pigmenten dar, kann aber nicht aus nachwachsenden Rohstoffen hergestellt werden. Darüber hinaus hinterlässt es im Vergleich zu anderen Produkten ein vergleichsweise fettigen Film auf der Haut (L. Rigano, R. Leporatti in Seifen, Öle, Fette, Wachse, 2004, 1/2, 12-22).

Letzterer Nachteil des Standes der Technik konnte teilweise von Estern des 1,3-Butandiols gelöst werden. So beschreibt EP0860164 B1 die Verwendung von Butylene Glycol Dicaprylat/Dicaprate welches unter dem Namen Dermofeel^{®} BGC vermarktet wird in Kombination mit dem UVB-Filter Ethylhexyl Triazon. Die Anwendungseigenschaften von Lichtschutzprodukten, welche Butylene Glycol Dicaprylat/Dicaprate enthalten werden in (L. Rigano, R. Leporatti in Seifen, Öle, Fette, Wachse, 2004, 1/2, 12-22) als vorteilhaft beschrieben. Ein weiterer Ester des 1,3-Butandiol ist das Butylene Glycol Cocoat, dessen Verwendung als Benetzmittel von Pigmenten in EP1807120 B1 beschrieben wird.

EP 1 859 779 beschreibt Lichtschutzzubereitungen, welche C12-C15 Alkylbenzoat, Butylenglycol Dicaprylat/Dicaprat und/oder Caprylic/Capric Triglycerid enthalten.

Aus der Gruppe der Diester von Dicarbonsäuren sei das Dibutyl Adipate genannt, welches unter dem Namen Cetiol B vielfältigen Einsatz findet.

Diese genannten Ester lösen in guter Weise die technischen Anforderungen an das Lösungs- und Dispergiervermögen von chemischen und physikalischen UV-Filtern, sie adressieren jedoch nicht die Anforderung, aus nachwachsenden Rohstoffen herstellbar zu sein. Sie lösen somit teilweise zwar die Nachteile des Standes der Technik, werden aber den ökologischen Anforderungen an moderne Kosmetikrohstoffe nicht in vollem Umfang gerecht.

Überraschenderweise und für den Fachmann nicht vorhersehbar wurden nun mit den genannten Estern, insbesondere Isopentyl Laurate (INCI: Isoamyl Laurate), Ölkomponenten identifiziert, welche aus nachwachsenden Rohstoffen hergestellt werden können und welche
- ein gutes Lösungsvermögen für die chemischen UV-Filter Ethylhexyl Triazon, Butyl Methoxydibenzoylmethan, Diethylamino Hydroxybenzoyl Hexyl Benzoate und Bis-Ethylhexyloxyphenol Methoxyphenyl Triazine besitzen,
- die Kristallisationsneigung der genannten UV-Filter auch in Gemischen mit anderen Ölkomponenten reduzieren,
- physikalische UV-Filter aus der Gruppe der oberflächlich unbehandelten oder modifizierten anorganischen Pigmente Titandioxid oder Zinkoxid hervorragend dispergieren und in der Formulierung stabilisieren,
- ein gutes Spreitvermögen besitzen und somit eine homogene Verteilung der genannten chemischen und physikalischen UV-Filter auf der Haut gewährleisten
und welche obendrein
- kosmetisch ansprechende Eigenschaften, wie ein schnelles Einziehvermögen, eine geringe Fettigkeit und eine geringe Filmbildung aufweisen,
und welche damit den oben genannten technischen Anforderungen für die genannten chemischen UV-Filter gerecht werden und den ökologischen und ökonomischen Nachteilen des Stands der Technik Abhilfe schaffen.

Die vorliegende Erfindung betrifft daher kosmetische und dermatologische Lichtschutzzubereitungen, insbesondere hautpflegende kosmetische und dermatologische Zubereitungen mit einem Gehalt an den genannten Estern wie bspw. Isoamyl Laurate und einem oder mehreren chemischen UV-Filtern aus der Gruppe:
- 2,4,6-Trianilino-p-(carbo-2'-ethylhexyl-1'oxy)-1,3,5-triazin (Ethylhexyl Triazon)
- 1-(4-tert-butylphenyl)-3-(4-methoxyphenyl)-propan-1,3-dion (Butyl Methoxydibenzoyl-methan)
- Bis-octyloxyphenol-methoxyphenyl-triazin (Bis-Ethylhexyloxyphenol Methoxyphenyl Triazine)
- 2-[-4-(Diethylamino)-2-hydroxybenzoyl] benzoesäurehexylester (Diethylamino Hydroxybenzoyl Hexyl Benzoate)
und / oder einem oder mehreren physikalischen UV-Filtern aus der Gruppe oberflächlich unbehandelter oder modifizierter anorganischer Pigment Titandioxid und / oder Zinkoxid.

Die erfindungsgemäßen Ölkomponenten für Sonnenschutzformulierungen können aus nachwachsenden Rohstoffen gewonnen werden, dabei den technischen Anforderungen gerecht werden und verfügen über sensorische Eigenschaften, welche die regelmäßige Verwendung des Sonnenschutzproduktes fördern.

Die Erfindung wird nachfolgend am Beispiel von Isoamyl Laurate als besonders bevorzugte Ausführungsform näher erläutert, ohne darauf beschränkt zu sein. Isoamyl Laurate ist aus der Veresterung von Isopentylalkohol und Laurinsäure herstellbar. Beide Rohstoffe können aus nachwachsenden Quellen gewonnen werden. Andere erfindungsgemäß verwendbare Isoamylester sind insbesondere die Ester von Isoamylalkohol mit Caprylsäure, Caprinsäure, Myristinsäure und Palmitinsäure.

Es war nun erstaunlich festzustellen, dass durch die erfindungsgemäße Zugabe von Isoamyl Laurate zu kosmetischen Formulierungen, welche die oben genannten UV-Filter enthalten, eine vergleichbare Menge dieser UV-Filter gelöst werden konnte, wie mit den Butylene Glycol Diestern bzw. dem C12-15 Alkylbenzoaten, die Formulierungen sich jedoch im Vergleich durch ein erheblich ansprechenderes Hautgefühl und eine gute Verteilung auf der Haut auszeichnen. Sie ziehen schnell in die Haut ein und hinterlassen keinen öligen oder klebrigen Film.

In erfindungsgemäßen Formulierungen können die genannten UV-Filter alleine oder in Kombination eingesetzt werden, um einen effektiven Breitbandschutz zu gewährleisten. Die Gesamtmenge an 2,4,6-Trianilino-p-(carbo-2'-ethylhexyl-1'oxy)-1,3,5-triazin (Ethylhexyl Triazon) wird dann vorteilhaft in einem Bereich von 0,1 % - 10 %, bevorzugt 0,5 - 6,0% gewählt, die Gesamtmenge an 1-(4-tert-butylphenyl)-3-(4-methoxyphenyl)-propan-1,3-dion (Butyl Methoxydibenzoyl-methan) wird vorteilhaft im Bereich 0,1 - 6,0 % bevorzugt 0,5 - 4,0% gewählt, die Gesamtmenge an Bis-octyloxyphenol-methoxyphenyl-triazin (Bis-Ethylhexyloxyphenol Methoxyphenyl Triazine) wird vorteilhaft im Bereich 0,1 % - 15 %, bevorzugt 0,5 - 10 % gewählt und die Menge 2-[-4-(Diethylamino)-2-hydroxybenzoyl] benzoesäurehexylester (Diethylamino Hydroxybenzoyl Hexyl Benzoate) wird im Bereich 0,1% - 10 %, bevorzugt 0,5 - 6% eingesetzt.

Kosmetische Lichtschutzprodukte, welche den Anforderungen von Naturkosmetik entsprechen sollen, dürfen wie oben erwähnt gemäß den formulierten Standards für derartige Produkte keine chemischen UV-Filter enthalten. Ihr UV-Schutz basiert ausschließlich auf der Verwendung physikalischer UV-Filter. Zur Realisierung eines ausreichenden Lichtschutzes sind dabei hohe Konzentrationen der verwendeten Titandioxid- und/oder Zinkoxidpigmente notwendig, welche sich in vielen Fällen negativ auf das Hautgefühl der Formulierung auswirken können. Es war nun überraschend festzustellen, dass Isoamyl Laurate auch hervorragend in der Lage war, sowohl oberflächlich unbehandelte als auch oberflächlich modifizierte Titandioxid und Zinkoxid Pigmente zu dispergieren und sich die erhaltenen erfindungsgemäßen Formulierungen im direkten Vergleich zu anderen Naturkosmetik konformen Ölkomponenten durch eine deutlich verbesserte Spreitfähigkeit und ein angenehmeres Hautgefühl auszeichneten.

Somit eignet sich die genannten Ester wie Isoamyl Laurate in hervorragender Weise sowohl zur Verwendung in klassischen Lichtschutzprodukten, deren Wirkung auf der Verwendung chemischer und physikalischer UV-Filter basiert, als auch zur Verwendung in zertifizierter Naturkosmetik deren Lichtschutz ausschließlich auf der Wirkung von anorganischen Pigmenten basiert.

Die Gesamtmenge an Isoamyl-Estern in erfindungsgemäßen Formulierungen beträgt 0,1 % - 50 %, vorzugsweise 0,5% - 20% bezogen auf das Gesamtgewicht der Formulierung. Es kann alleine aber selbstverständlich auch in Kombination mit anderen Ölkomponenten zum Lösen der genannten UV-Filter, wie z.B. C12-15 Alkylbenzoat, Butylene Glycol Dicaprylat / Dicaprat oder Dibutyladipat verwendet werden. Sämtliche Prozentangaben in den Patentansprüchen und der Beschreibung sind Gewichtsprozente, sofern nicht anders angegeben.

Die erfindungsgemäßen Sonnenschutzformulierungen können wie üblich zusammengesetzt sein und dem kosmetischen und/oder dermatologischen Lichtschutz aber auch zur Behandlung, Pflege und Reinigung der Haut und / oder Haare dienen oder auch als Produkt der dekorativen Kosmetik.

Sie können kosmetische Hilfsstoffe enthalten, wie sie üblicherweise in solchen Formulierungen verwendet werden wie beispielsweise weitere Ölkörper, Emulgatoren, Perlglanzwachse, Konsistenzgeber, Verdickungsmittel, Überfettungsmittel, Stabilisatoren, Polymere, Siliconverbindungen Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektrepellentien, Selbstbräuner, Tyrosininhibitoren (Depigmentierungsmittel), Hydrotrope, Solubilisatoren, Konservierungsmittel, Parfümöle, Farbstoffe etc., die nachstehend exemplarisch aufgelistet sind.

### Ölkörper

Körperpflegemittel, wie Cremes, Lotionen und Milchen, enthalten üblicherweise eine Reihe weiterer Ölkörper und Emollients, die dazu beitragen die sensorischen Eigenschaften weiter zu optimieren. Die Ölkörper sind üblicherweise in einer Gesamtmenge von 0,1 - 50 Gew.-%, vorzugsweise 5 - 25 Gew.-% und insbesondere 5 - 15 Gew.% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, CVetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isosterylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat, Behenylmyristat, Behenylpalmitat, Behenylpalmitat, Behenylstearat, Behenylisostearat, Behenyloleat, Behenylbehenat, Erucylmyristat, Erucylpalmitat, Erucylstearat, Erucylisostearat, Erucyloleat, Erucylbehenat und Erucylerucat in Betracht. Daneben eigenen sich auch Ester von linearen C₆-C₂₂-Fettsäuren mit unverzweigten oder verzweigten mehrwertigen Alkoholen wie z.B. Butylene Glycol Dicaprylat/Dicaprat (Dermofeel^{®} BGC) oder Ester des 2-Methyl-1,3-propandiols, des 1,1,1-Trimethylolethans oder 1,1,1-Trimethylolpropans. Desweiteren eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis von C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv^{®} TN), lineare oder verzweigte, symmetrische oder unsymmetrische Dialkylether mit 6 bis 22 Kohlenstoffatomen pro Alkylgruppe, wie z. B. Dicaprylyl Ether (Cetiol^{®} OE), Ringöffnungsprodukte von epoxidierten Fettsäureestern mit Polyolen, Siliconöle (Cyclomethicone, Sliciummethicontypen u. a.) und/oder aliphatische bzw. naphthenische Kohlenwasserstoffe, wie z. B. Squalan, Squalen oder Dialkylcyclohexane.

### Emulgatoren

Als Emulgatoren kommen beispielsweise nicht-ionogene Tenside aus mindestens einer der folgenden Gruppen in Frage:
- Anlagerungsprodukte von 2 bis 30 Mol Ethylenoxid und/oder 0 bis 5 Mol Propylenoxid an linear Fettalkohole mit 8 bis 22 C-Atomen, an Fettsäuren mit 1 bis 22 C-Atomen, an Alkylphenole mit 8 bis 15 C-Atomen in der Alkylgruppe sowie Alkylamine mit 8 bis 22 Kohlenstoffatomen im Alkylrest
- Alkyloligoglykoside mit 8 bis 22 Kohlenstoffatomen im Alkylrest und deren ethoxylierte Analoga
- Anlagerungsprodukte von 1 bis 15 Mol Ethylenoxid an Ricinusöl und/oder gehärtetes Ricinusöl
- Anlagerungsprodukte von 15 bis 60 Mol Ethylenoxid an Ricinusöl und/oder gehärtetes Ricinusöl
- Partialester von Glycerin und/oder Sorbitan mit ungesättigten, linearen oder gesättigten, verzweigten Fettsäuren mit 12 bis 22 Kohlenstoffatomenund/oder Hydroxyarbonsäuren mit 3 bis 18 Kohlenstoffatomen sowie deren Addukte mit 1 bis 30 Mol Ethylenoxid.
- Partialester von Polyglycerin (durchschnittlicher Eigenkondenstationsgrad 2 bis 8), Polyethylenglycol (Molekulargewicht 400 bis 5000), Trimethylolpropan, Pentaerythrit, Zuckeralkoholen (z. B. Sorbit), Alkylglucosiden (z. B. Methylglucosid, Butylglucosid, Laurylglucosid) sowie Polyglucosiden (z. B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin
- Mono-, Di- und Trialkylphosphate sowie Mono-, Di- und/oder Tri-PEG-alkylphosphate und deren Salze
- Wollwachsalkohole
- Polysiloxan-Polyalkyl-Polyether-Copolymere bzw. Entsprechende Derivate
- Block-Copolymere z. B. Polyethylenglycol-30-Dipolyhydroxystearat
- Polymeremulgatoren, z. B. Pemulen-Typen (TR-1, TR-2) von Goodrich sowie
- Polyalkylenglycole

### - Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und diester von Anlagerungsprodukten von Ethylenoxid an Glycerin sind als Rückfettungsmittel für kosmetische Zubereitungen bekannt.

### - Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansequioleat, Sorbitandioleat, Sorbitantrioleat, Sorbitanmonoerucat, Sorbitansesquierucat, Sorbitandierucat, Sorbitantrierucat, Sorbitanmonoricinolat, Sorbitansesquiricinolat, Sorbitandiricinolat, Sorbitantriricinolat, Sorbitanmonohydroxystearat, Sorbitanmonotartrat, Sorbitansesquitartrat, Sorbitanditartrat, Sorbitantritartrat, Sorbitanmonocitrat, Sorbitansesquitartrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### - Polyglycerinester

Typische Beispiele für geeignet Polyglycerinester sind Polyglyceryl-3 Stearate (Dermofeel^{®} PS), Polyglycerin-3-Palmitate (Dermofeel^{®} PP), Polyglyceryl-6 Caprylate (Dermofeel^{®} G 6 CY), Polyglyceryl-10 Laurate (Dermofeel^{®} G 10 L), Polyglyceryl-2-Laurate (Dermofeel^{®} G 2 L), Polyglyceryl-3-Laurate (Dermofeel^{®} G 3 L), Polyglyceryl-5-Laurate (Dermofeel^{®} G 5 L), Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} G1 34), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Dermofeel^{®} PR) Polyglyceryl Dimerate Isostearate sowie deren Gemische. Beispiele für weitere geignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### - Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäure mit 12 bis 22 Kohlenstoffatomen, wie beispielsweise Azelainsäure oder Sebacinsäure.

### - Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-NN-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinate, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinate. Besonders geeignet ist das unter der CTFA-Bezeichnung Cocoamidopropyl Betaine bekannte Fettsäureamid-Derivat. Ebenfalls geeignete Emulgatoren sind ampholytische Tenside. Unter ampholyytischen Tensiden werden olche oberflächenaktiven Verbindungen verstanden, die außer einer C_{8/18}-Alkyl- oder Acylgruppe im Molekül mindestens eine freie Aminogruppe und mindestens eine -COOH oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren N-Alkyliminidipropionäuren, N-Hydroxyethyl-N-alkylamidopropylglycin, N-Alkyltaurine, N-Alkylsarcosine, 2-Alkylaminopropionsäuren und Alkylminoessigsäuren mit jeweilsetwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ des Esterquats, vorzugsweise methylquaternierte Difettsäuretriethanolaminester-Salze, besonders bevorzugt sind.

### Fett und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d.h. feste oder flüssige, pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwchse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher häufig auch als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierten Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 - 15 Kohlenstoffatomen und 2 bis 10 Hydroxylgruppen sowie deren Mischungen.

### Konsistenzgeber und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Bettracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (Hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und diester von Fettsäuren, Polyacrylate (z. B. Carbopole^{®} und Permulene-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z. B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit geeigneter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z. B. Magnesium-, Aluminium- und/oder Zinkstearat bzw. ricinoleat eingesetzt werden.

### Polymere

Geeignete kationische Polymere sind beispielsweise kationische Cellulosederivate, wie z. B. eine quaternisierte Hydroxyethylcellulose, die unter der Bezeichnung Polymer JR 400^{®} von Amerchol erhältlich ist, kationische Stärke, wie z.B. quaternisierte Stärken, die unter dem Markennamen Amylomer^{®} und symbio^{®}quat von Dr. Straetmans erhältlich sind, Copolymere von Diallylammoniumsalzen und Acrylamiden, quaternierte Vinylpyrrolidon/Vinilimidazol-Polymere, wie z. B. Luviquat^{®} (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z. B. A-modimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlösliche Polymere, kationische Chitinderivate wie beispielsweise quaterniertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, kationisches Guar-Gum, wie z. B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/AcrylatCopolymere, Octylacrylamid/Methylmethacrylat/tert.-Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Neben den erfindungsgemäßen UV-Filtern können weitere UV-Filter in den erfindungsgemäßen Formulierungen vorliegen. UV-B-Filter können öllöslich oder wasserlöslich sein. Als öllösliche Substanzen sind z.B. zu nennen:
- 3-Benzylidencampher bzw. 3-3-Benzylidennorcampher und dessen Derivate, z.B. 3-(4-Methylbenzyliden)campher wie in der Patentschrift EP 0693471 B1 beschrieben.
- 4-Aminobenzoesäurederivate, vorzugsweise 4-(Dimethylamino)benzoesäure-2-ethylhexylester, 4-(Dimethylamino)benzoesäure-octylester und 4-(Dimethylamino)benzoesäure-2-amylester.
- Ester der Zimtsäure, vorzugsweise 4-Methoxyzimtsäure-2-ethylhexylester, 4-Methoxyzimtsäure-propylester, 4-Methoxyzimtsäure-isoamylester oder 2-Cyano-3,3-phenylzimtsäure-2-ethylhexylester (Octocrylene)
- Ester der Salicylsäure, vorzugsweise Salicylsäure-2-ethylhexylester, Salicylsäure-4-isopropylbenzylester oder Salicylsäurehomomenthylester
- Derivate des Bezophenons, vorzugsweise 2-Hydroxy-4-methoxybezophenon, 2-Hydroxy-4'-methoxybezophenon, 2,2'-Dihydoxy-4-methoxybenzophenon
- Ester der Benzalmalonsäure, vorzugsweise 4-Methoxybenzmalonsäure-di-2-ethylhexylester
- Triazinderivate wie z.B. Dioctyl Butamido Triazon (U-vasorb HEB)
- Ketotricyclo(5.2.1.0)decan-Derivate, wie in der EP 0694521 B1 beschrieben.

Als wasserlösliche Substanzen kommen in Frage:
- 2-Phenylbenzimidazol-5-sulfonsäure und deren Alkali-, Erdalkali-, Ammonium-, Alkylammonium, Alkanolammonium- und Glucammoniumsalze
- Sulfonsäurederivate von Benzophenon, vorzugsweise 2-Hydroxy-4-methoxybenzophenon-5-sulfonsäure und ihre Salze
- Sulfonsäurederivate des Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-Phenyl-3-(4'isopropylphenyl)-propan-1,3-dion sowie Enaminverbindungen, wie beschrieben in der DE 19712033 A1. Die UV-A- und UV-B- Filter können selbstverständlich auch in Mischungen eingesetzt werden. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium, Alkanolammonium- und Glucammonumsalze kombiniert.

Neben den genannten löslichen Stoffen kommen für diesen Zweck auch unlösliche Lichtschutzpigmente, nämlich feindisperse Metalloxide bzw. salze in Frage. Beispiele für geeignete Metalloxide sind insbesondere Titandioxid und Zinkoxid und daneben Oxide des Eisens, Zirkoniums, Siliziums, Mangans, Aluminiums und Cers sowie deren Gemische. Als salze können Silicate (Talk), Bariumsulfat oder Zinkstearat eingesetzt werden. Die Oxide und Salze werden in Form der Pigmente für hautpflegende und hautschützende Emulsionen du dekorative Kosmetik verwendet. Die Partikel sollen dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobisiert vorliegen. Typische Beispiele sind gecoatete Titandioxide, wie z.B. Titandioxid T 805 der Fa. Degussa oder Eusolex T 2000 der Fa. Merck. Als hydrophobe Coatingmittel kommen dabei vor allem Silicone und dabei speziell Trialkoxyoctylsilane oder Simethicone in Frage. In Sonnenschutzmitteln werden bevorzugt so genannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn schädliche UV-Strahlung in die Haut eindringt. Typische Beispiele sind hierfür Aminosäure(z.B. Histidin, Tyrosin oder Tryptophan) und deren Derivate, Imidazole (z.B. Urocaninsäure) und deren Derivate, Peptide wie D,L-Carnosin, D-Carnosin, L-Carnosin und deren Derivate, Carotinoide, Carotine (z.B. α-Carotin, β-Carotin, Lycopin) und deren Derivate, Liponsäuren und deren Derivate (z.B. Dihydroliponsäure), Aurothioglucose, Propylthiouracil und andere Thiole (z.B. Thioredoxin, Glutathion, Cystein, Cystin, Cysteamin un deren Glycolsyl-, N-Acetyl-, Mehyl-, Ethyl-, Propyl-, Amyl-, Butyl-, Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximin, Homocysteinsulfoximin, Butioninsulfoine, Penta-, Hexa-, Heptathioninsulfoximin) in sehr geringen verträglichen Dosierungen (z.B. pmol bis mol), ferner Komplexbildener wie z.B. a-Hydroxyfettsäuren, Palmitinsäure, Phytinsäure, Lactoferrin), a-Hydroxysäurem (z.B. Zitronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbyl Palmitat, Magnesium Ascorbylphosphat, Ascobylacetat), Tocopherole (z.B. α-Tocopherol, β-Tocopherol, γ-Tocopheol δ-Tocopherol) und deren Derivate (Tocopherly Acetat), Vitamin A und Derivate (Vitamin A Palmitat) soweie Koniferylbenzoat des Benzoeharzes, Rutinsäure und deren derivate, α-Glycosylrutin, Ferulasäure, Furfurylidengluticol, Carnosin, Butylhydrxytoluol, Butylhydroxyanisol, Nordihydroguarjakharzsäure, Nordihydroguarjaretsäure, Trihydroxybutyrophenon, Harnsäure und deren Derivate, Mannose und deren Derivate, Speroxid-Dismutase, Zink und dessen Derivate (ZnO, ZnSO₄), Selen und dessen Derivate (z.B. Selen-Methionin), Stilbene und deren Derivate (z.B. Stilbenoxid, trans-Stilbenoxid) und die errfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Deodorantien und keimhemmende Mittel

Kosmetische Deodorantien (Desodorantien)wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### - Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DGMC), Salicylsäure-N-alkylamide wie z. B. Salicylsäure-n-octylamid oder Salicylsäure-n-decylamid.

### - Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### - Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfumöle, die zusätzlich zu ihrer Funktion als Geruchsüberdecker den Deodorantien ihre jeweilige Duftnote verleihen. Als Parfumöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropinonat, und Benzylsalicylat. Zu den Ethern zähln beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, -Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Couer, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen eingesetzt.

### - Antitranspirantien

Antitranspirantien (Antiperspipantien) reduzieren durch Beeinflussung der Aktivität der ekkrinen Schweißdrüsen die Schweißbildung und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe
- Ölkomponenten
- nichtionische Emulgatoren
- Coemulgatoren
- Konsistenzgeber
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat,Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringen Mengen enthalten sein. Solche öllöslichen Hilfsmittel können z. B. sein:
- entzündungshemmende, hautschützende oder wohlriechende ätherische Öle
- synthetische hautschützende Wirkstoffe und/oder
- öllösliche Parfumöle

Übliche wasserlösliche Zusätze sind z. B. Konservierungsmittel, wasserlösliche Duftstoffe, pH-Wert-Stellmittel, z. B. Puffergemische, wasserlösliche Verdickungsmittel, z. B. wasserlösliche natürliche oder synthetische Polymere wie z. B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-und Stärkederivate, Kollagen, Hyaluronsäure bzw. deren Salze und ähnliche Verbindungen.

### Quellmittel

Als Quellmittel für wässrige Phasen können Montmorillonite, Clay Mineralstoffe, Pemulen sowie alkyl-modifizierte Carbopoltypen (Goodrich) dienen. Weitere geeignete Polymere bzw. Quellmittel können der Übersicht von R. Lochhead in Cosm. Toil. 108, 95 (1993) entnommen werden.

### Insekt-Repellentien

Als Insekt-Repellentien kommen beispielsweise N,N-Diethyl-m-toluamid, 1,2-Pentandiol oder 3-(N-n-Butyl-N-acetyl-amino)-propionic aid ethyl ester), welches unter der Bezeichnung Insect Repellent^{®} 3535 von der Merck KGaA vertrieben wird, sowie Butylacetylaminopropionate in Frage.

### Selbstbräuner und Depigmentierungsmittel

Als Selbstbräuner eignet sich Dihydroxyaceton und Erythrulose. Als Tyrosininhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Typische Beispiele sind:
- Glycerin
- Alkylenglycole, wie beispielsweise Ethylenglycol, Diethylenglycol, Propylenglycol, Butylenglycol, Pentylenlycol, Hexylenglycol, 1,2-Hexandiol und Caprylyl Glycol sowie Polyethylenglycole mit einem durchschnittlichen Molekulargewicht von 100 bis 1.000 Dalton.
- technische Oligoglyceringemische mit einem Eigenkondensationsgrad von 1,5 bis 10 wie etwa technische Diglyceringemische mit einem Diglyceringehalt von 40 bis 50 Gew.-%
- Methylolverbindungen, wie insbesondere Trimethylolethan, Trimethylolpropan, Trimethylolbutan, Pentaerythrit und Dipentarythrit
- Niedriglkylglucoside, insbesondere solche mit 1 bis 8 Kohlenstoffen im Alkylrest, wie beispielsweise Methyl- und Butylglucosid
- Zuckeralkohole mit 5 bis 12 Kohlenstoffatomen, wie beispiele Sorbit oder Mannit
- Zucker mit 5 bis 12 Kohlenstoffatomen, wie beispielsweise Glucose oder Saccharose
- Aminozucker, wie beispielsweise Glucamin
- Dialkoholamine, wie Diethanolamin oder 2-Amino-1,3-propandiol

### Konservierungsmittel

Als Konservierungsmittel eignen sich beispielsweise Phenoxyethanol, Parabene, Pentandiol oder Sorbinsäure sowie die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange),Wurzeln (Macis, Angelica, Sellerie, Kardamom, Costus, Iris, Calmus), Hölzern (Pinien- Sandel- Guajak-, Zedern-, Rosenholz), Kräutern und Grsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone, α-Isomethylionon und Methylcedrylketon, zu den AlkoholenCitronellol, Eugenol, Isoeugenol, Geraniol, Linalool, Phenylethylalkohol, 3 -Phenyl-1-propanol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydrmyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambraxon, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Auch Duft- und Aromastoffe aus der Gruppe der organischen Säuren, wie z.B. p-Anissäure, 4-Oxopentansäure oder Zimtsäure können zur Beduftung von erfindungsgemäßen Formulierungen herangezogen werden. Letztere können darüber hinaus zur mikrobiologischen Stabilisierung der Formulierungen beitragen

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I. 42051), Indigotin (C.I. 73015), Chlorophyllin (C.I. 75810), Chinolongelb (C.I. 47005), Titandioxid (C.I. 77891), Indanthrenblau RS (C.I. 69800) und Krapplack (C.I. 58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Drei Ausführungsbeispiele der Erfindung werden nachfolgend erläutert.

### 1. PEG-frei O/W - Sonnenschutzlotion

| **Phase** | **Rohstoffe** | **INCI** | **Lieferant** | **%** |
|---|---|---|---|---|
| A | Deionised Water | Aqua | | ad. 100 |
| | Dermofeel PA-3 | Sodium Phytate, Aqua | Dr. Straetmans | 0,10 |
| | Glycerol | Glycerin | | 3,50 |
| | Dermosoft LP | Caprylyl Glycol, Glycerin, Glyceryl Caprylate, Phenyl-propanol | Dr. Straetmans | 1,00 |
| A1 | Cosmedia SP | Sodium Polyacrylate | Cognis | 0,20 |
| | Dermofeel GSC | Glyceryl Stearate Citrate | Dr. Straetmans | 2,50 |
| B | Dermofeel sensolv | Isoamyl Laurate | Dr. Straetmans | 8,00 |
| | Cutina GMS | Glyceryl Stearate | Cognis | 2,00 |
| | Lanette O | Cetearayl Alcohol | Cognis | 1,00 |
| | Cetiol B | Dibutyl Adipate | Cognis | 5,00 |
| | Neo Heliopan AV | Ethylhexyl methoxycinnamate | Symrise | 5,00 |
| | Parsol 1789 | Butyl Methoxydibenzoyl-methane | DSM | 3,00 |
| | Tinosorb S | Bis-Ethylhexyloxyphenol methoxyphenyl Triazione | Ciba | 3,00 |
| | Uvinul T150 | Ethylhexyl Triazone | BASF | 3, 00 |
| C | DC 246 Fluid | Cyclohexasiloxane, Cyclopentasiloxane | Dow Corning | 2,00 |
| D | Parfümöl Sugar Ray DH10316 | Parfum | Symrise | 0,20 |
| | Tinosorb M | Methylene Bis-Benzotriazoyl Tetramethylbutylphenol | Ciba | 4,00 |
| | Sodium Hydroxid | Sodium Hydroxid | | 0,13 |
| Herstellung: Phase A auf 80°C erwärmen A1 hinzufügen und mit Ultra Turrax homogen dispergieren. Phase B auf 78°C erwärmen. Phase B zu Phase A unter Rühren, anschließend ca. 1 min mit Ultra Turrax homogenisieren. Abkühlen auf ca. 60°C und Phase C hinzufügen. Weiter unter Rühren abkühlen bis auf Raumtemperatur und Phase D zufügen. | | | | |

### 2. Natural Sun W/O

| **Phase** | **Rohstoffe** | **INCI** | **Lieferant** | **%** |
|---|---|---|---|---|
| A | Tap Water | Aqua | | ad. 100 |
| | Glycerin | Glycerin | | 7,00 |
| | Zinksulfat Heptahydrat | Zinc Sulfate | Merck | 1, 00 |
| B | Dermofeel PR | Polyglyceryl-3 Polyricinoleate | Dr. Straetmans | 5,00 |
| | Dermosoft GMC | Glyceryl Caprate | Dr. Straetmans | 0, 50 |
| | Dermofeel PO | Glyceryl Oleate | Dr. Straetmans | 1,00 |
| | Dermofeel sensolv | Isoamyl Laurate | Dr. Straetmans | 10,00 |
| | Dermofeel MCT | Tricaprylin | Dr. Straetmans | 15,00 |
| | Cutina HR | Hydrogenated Castor Oil | | 0,25 |
| | Bienenwachs 8104, weiß, rein | Cera Alba | Kahl & Co | 0,25 |
| | Emprove | Magnesium Stearate | Merck | 0,50 |
| | alpha Bisabolot, nat. | Bisabolol | GfN | 0,10 |
| | Dermofeel Toco 70 non-GMO | Tocopherol | Dr. Straetmans | 0,20 |
| B1 | Zinc Oxide | Zinc Oxide | BASF | 5,00 |
| | Uv-Titan M160 | Titanium Dioxide | Kemira | 4,6 |
| C | Parfümöl Sugar Ray DH10316 | Parfum | Frey & Lau | 0,2 |
| **Herstellung:** Phase A auf 80°C erwärmen. Phase B auf 80°C erwärmen. Phase B1 in Phase B dispergieren. Phase A langsam unter Rühren zu Phase B geben, anschließend für 2-5 Min. mit dem Ultra Turrax homogenisieren. Unter Rühren auf 32°C abkühlen und Phase C hinzufügen. Weiter auf Raumtemperatur abkühlen. | | | | |

### 3. Anti Ageing Light Day Care Cream SPF 10

| **Phase** | **Rohstoffe** | **INCI** | **Lieferant** | **%** |
|---|---|---|---|---|
| A | Deionised Water | Aqua | | ad. 100 |
| | Glycerol | Glycerin | | 3,00 |
| | Dermofeel PA-3 | Sodium Phytate | Dr. Straetmans | 0,10 |
| | Dermosoft LP | Caprylyl Glycol, Glycerin, Glyceryl Caprylate, Phenylpropanol | Dr. Straetmans | 0,80 |
| A1 | Cosmedia SP | Sodium Polyacrylate | Cognis | 0,20 |
| | Keltrol RD | Xanthan Gum | CP Kelco | 0,30 |
| B | Symbiomuls GC | Glyceryl Stearate Citrate, Cetearyl Alcohol, Glyceryl Caprylate | Dr. Straetmans | 5,00 |
| | Cetiol SB 45 | Shea Butter | Cognis | 2,50 |
| | Phytosqualan | Squalane | | 5,00 |
| | Dermofeel sensolv | Isoamyl Laurate | Dr. Straetmans | 8,00 |
| | DC 345 | Cyclopentasiloxane, Cyclohexasiloxane | Dow Coming | 3,00 |
| | DC 200 | Dimethicone | Dow Coming | 1,00 |
| | Uvinul A plus | Diethylamino Hydroxybenzoyl Hexyl Benzoate | BASF | 2,80 |
| | Uvinul MC 80 | Ethylhexyl methoxycinnamate | BASF | 5,20 |
| | Dermofeel E74 A | Tocopherol Acetate | Dr. Straetmans | 0, 50 |
| C | Sculptessence | Water, Glycerin, Linum Usitatissimum Seed Extract | Lucas Meyer Cosmetics | 4,00 |
| D | Mamaku Vital esence PC Panther (flowery, fruity, fresh) Sensitizer free P0261246 | Water, Cyathea medullaris leaf extract Parfum | Lucas Meyer Cosmetics Frey & Lau | 2,00 0,30 |
| Sodium Hydroxid Sodium Hydroxid 0,25 Herstellung: Phase A auf 78°C erwärmen und Phase A1 eindispergieren. Phase B auf 78°C erwärmen. Unter Rühren Phase B zu Phase A hinzufügen und anschließend für 1-2 Minuten mit dem Ultra Turrax homogenisieren. Emulsion unter mittlerem Rühren abkühlen und bei unter 40°C Phase C und Phase D hinzufügen. | | | | |

## Patentansprüche

1. Kosmetische oder dermatologische Zubereitung, **dadurch gekennzeichnet, dass** sie enthält:
a) einen oder mehrere chemische Lichtschutzfilter ausgewählt aus der Gruppe bestehend aus 2,4,6-Trianilino-p-(carbo-2'-ethylhexyl-1'oxy)-1,3,5-triazin (Ethylhexyl Triazon), 1-(4-tert-butylphenyl)-3-(4-methoxyphenyl)-propan-1,3-dion (Butyl Methoxydibenzoyl-methan), Bis-octyloxyphenol-methoxyphenyl-triazin (Bis-Ethylhexyloxyphenol Methoxyphenyl Triazin) und 2-[4-(Diethylamino)-2-hydroxybenzoyl] benzoesäurehexylester (Diethylamino Hydroxybenzoyl Hexyl Benzoate); und/oder einen oder mehrere Lichtschutzfilter ausgewählt aus der Gruppe bestehend aus oberflächlich behandelten oder unbehandelten Pigmenten des Titandioxid oder Zinkoxid;
b) wenigstens einen Ester einer C8-C16-Fettsäure und Isoamylalkohol.

2. Zubereitung nach Anspruch 1, **dadurch gekennzeichnet, dass** die Fettsäure Laurinsäure ist.

3. Zubereitung nach Anspruch 1 oder 2, **dadurch gekennzeichnet, dass** sie den Ester einer C8-C16-Fettsäure und Isoamylalkohol in einer Konzentration von 0,1 - 50 % bevorzugt 0,5 - 20 % enthält.

4. Zubereitung nach einem der Ansprüche 1 bis 3, **dadurch gekennzeichnet, dass** sie 2,4,6-Trianilino-p-(carbo-2'-ethylhexyl-1'oxy)-1,3,5-triazin (Ethylhexyl Triazon) in einer Konzentration von 0,1 % - 10 %, bevorzugt 0,5 % - 6,0 % enthält.

5. Zubereitung nach einem der Ansprüche 1 bis 3, **dadurch gekennzeichnet, dass** sie 1-(4-tert-butylphenyl)-3-(4-methoxyphenyl)-propan-1,3-dion (Butyl Methoxydibenzoylmethan) in einer Konzentration von 0,1 % - 6,0 %, bevorzugt 0,5 % - 4,0 % enthält.

6. Zubereitung nach einem der Ansprüche 1 bis 3, **dadurch gekennzeichnet, dass** sie Bis-octyloxyphenol-methoxyphenyl-triazin (Bis-Ethylhexyloxyphenol Methoxyphenyl Triazin) in einer Konzentration von 0,1 % - 15,0 %, bevorzugt 0,5 % - 10,0 % enthält.

7. Zubereitung nach einem der Ansprüche 1 bis 3, **dadurch gekennzeichnet, dass** sie 2-[4-(Diethylamino)-2-hydroxybenzoyl] benzoesäurehexylester (Diethylamino Hydroxybenzoyl Hexyl Benzoate) in einer Konzentration von 0,1% - 10 %, bevorzugt 0,5 - 6% enthält.

8. Zubereitung nach einem der Ansprüche 1 bis 7, **dadurch gekennzeichnet, dass** sie 2 - 10% oberflächlich unmodifizierte oder modifizierte Titandioxidpigmente enthält.

9. Zubereitung nach einem der Ansprüche 1 bis 7, **dadurch gekennzeichnet, dass** sie 2 - 10% oberflächlich unmodifizierte oder modifizierte Zinkoxidpigmente enthält.

## Claims

1. Cosmetic or dermatological preparation, **characterised in that** it contains:
a) one or more chemical sun protection filters selected from the group consisting of 2,4,6-trianilino-p-(carbo-2'-ethylhexyl-1'oxy)-1,3,5-triazine (ethylhexyl triazone), 1-(4-tert-butylphenyl)-3-(4-methoxyphenyl)-propane-1,3-dion (butyl methoxydibenzoylmethane), bis-octyloxyphenol-methoxyphenyl-triazine (bis-ethylhexyloxyphenol methoxyphenyl triazine) and 2-[4-(diethylamino)-2-hydroxybenzoyl] benzoic acid hexyl ester (diethylamino hydroxybenzoyl hexyl benzoate); and/or one or more sun protection filters selected from the group consisting of superficially treated or untreated pigments of titanium dioxide or zinc oxide;
b) at least one ester of a C8-C16 fatty acid and isoamyl alcohol.

2. Preparation according to claim 1, **characterised in that** the fatty acid is lauric acid.

3. Preparation according to either claim 1 or claim 2, **characterised in that** it contains the ester of a C8-C16 fatty acid and isoamyl alcohol in a concentration of from 0.1 % to 50 %, preferably of from 0.5 % to 20 %.

4. Preparation according to any of claims 1 to 3, **characterised in that** it contains 2,4,6-trianilino-p-(carbo-2'-ethylhexyl-1'oxy)-1,3,5-triazine (ethylhexyl triazone) in a concentration of from 0.1 % to 10 %, preferably of from 0.5 % to 6.0 %.

5. Preparation according to any of claims 1 to 3, **characterised in that** it contains 1-(4-tert-butylphenyl)-3-(4-methoxyphenyl)-propane-1,3-dion (butyl methoxydibenzoylmethane) in a concentration of from 0.1 % to 6.0 %, preferably of from 0.5 % to 4.0 %.

6. Preparation according to any of claims 1 to 3, **characterised in that** it contains bis-octyloxyphenol-methoxyphenyl-triazine (bis-ethylhexyloxyphenol methoxyphenyl triazine) in a concentration of from 0.1 % to 15.0 %, preferably of from 0.5 % to 10.0 %.

7. Preparation according to any of claims 1 to 3, **characterised in that** it contains 2-[4-(diethylamino)-2-hydroxybenzoyl] benzoic acid hexyl ester (diethylamino hydroxybenzoyl hexyl benzoate) in a concentration of from 0.1 % to 10 %, preferably of from 0.5 % to 6 %.

8. Preparation according to any of claims 1 to 7, **characterised in that** it contains 2 % to 10 % superficially unmodified or modified titanium dioxide pigments.

9. Preparation according to any of claims 1 to 7, **characterised in that** it contains 2 % to 10 % superficially unmodified or modified zinc oxide pigments.

## Revendications

1. Préparation cosmétique ou dermatologique, **caractérisée en ce qu'**elle contient
a) un ou plusieurs filtres antisolaires chimiques, choisis parmi le groupe constitué de la 2,4,6-trianilino-p(carbo-2'-éthylhexyl-1'-oxy)-1,3,5-triazine (éthylhexyl triazone), de la 1-(4-tert-butylphényl)-3-(4-méthoxyphényl)-propano-1,3-dione (butyl méthoxydibenzoyl-méthane), de la bisoctyloxyphénol-méthoxyphényl-triazine (bis-éthylhexyloxyphénol méthoxyphényle triazine) et de l'ester hexylique de l'acide 2-[4-(diéthylamino)-2-hydroxybenzoyl] benzoïque (diéthylamino hydroxybenzoyl hexyl benzoate); et/ou un ou plusieurs filtres antisolaires choisi parmi le groupe constitué de pigments de dioxyde de titane ou d'oxyde de zinc traités en surface ou non traités ;
b) au moins un ester d'un acide gras en C8 à C16 et de l'alcool isoamylique.

2. Préparation selon la revendication 1, **caractérisée en ce que** l'acide gras est de l'acide laurique.

3. Préparation selon la revendication 1 ou 2, **caractérisée en ce que** qu'elle contient l'ester d'un acide gras en C8 à C16 et de l'alcool isoamylique en une concentration de 0,1 à 50 % de préférence de 0,5 à 20 %.

4. Préparation selon l'une quelconque des revendications 1 à 3, **caractérisée en ce qu'**elle contient de la 2,4,6-trianilino-p-(carbo-2'-éthylhexyl-1'-oxy)-1,3,5-triazine (éthylhexyl triazone) en une concentration de 0,1 % à 10 %, de préférence de 0,5 % à 6,0 %

5. Préparation selon l'une quelconque des revendications 1 à 3, **caractérisée en ce qu'**elle contient de la 1-(4-tert-butylphényl)-3-(4-méthoxyphényl)-propano-1,3-dione (butyl méthoxydibenzoylméthane) en une concentration de 0,1 % à 6,0 ° %, de préférence de 0,5 % à 4,0 %.

6. Préparation selon l'une quelconque des revendications 1 à 3, **caractérisée en ce qu'**elle contient de la bis-octyloxyphénol-méthoxyphényl-triazine (bis-éthylhexyloxyphénol méthoxyphényl triazine) en une concentration de 0,1 % à 15,0 %, de préférence de 0,5 % à 10,0 %.

7. Préparation selon l'une quelconque des revendications 1 à 3, **caractérisée en ce qu'**elle contient de l'ester hexylique de l'acide 2-[4-(diéthylamino)-2-hydroxybenzoyl] benzoïque (diéthylamino hydroxybenzoyl hexyl benzoate) en une concentration de 0,1% à 10 %, de préférence de 0,5 à 6%.

8. Préparation selon l'une quelconque des revendications 1 à 7, **caractérisée en ce qu'**elle contient de 2 à 10 % de pigments de dioxyde de titane non modifiés ou modifiés en surface.

9. Préparation selon l'une quelconque des revendications 1 à 7, **caractérisée en ce qu'**elle contient de 2 à 10 % de pigments d'oxyde de zinc non modifiés ou modifiés en surface.
